# FASCICULE DE BREVET EUROPEEN

(11) **EP 2 220 209 B1**
(45) Date de publication et mention de la délivrance du brevet: **21.04.2021**
(21) Numéro de dépôt: 08872580.9
(22) Date de dépôt: 08.12.2008
(51) Int. Cl.: B01F 7/16, C12M 1/33

(54) **DISPOSITIF DE BROYAGE D'UN ÉCHANTILLON BIOLOGIQUE**
VORRICHTUNG ZUR ZERKLEINERUNG VON BIOLIGISCHEN PROBEN
DEVICE FOR GRINDING A BIOLOGICAL SAMPLE

(30) Priorité: 18.12.2007 FR 0708835
(43) Date de publication de la demande: 25.08.2010
(73) Titulaire: Bertin Technologies, 78180 Montigny-le-Bretonneux (FR)
(72) Inventeur: BOUGY, Jean-Jacques, F-78890 Garancières (FR)
(74) Mandataire: Decobert, Jean-Pascal
(86) Numéro de dépôt international: PCT/FR2008/001705
(87) Numéro de publication internationale: WO 2009/103868

(56) Documents cités:
- EP-A- 0 590 219
- WO-A-99/02958
- WO-A-2004/035191
- WO-A-2005/047866
- WO-A-2006/076819
- CA-A1- 2 622 138
- DE-B- 1 212 248
- GB-A- 1 356 794
- US-A- 2 678 809
- US-A1- 2004 035 964
- US-B1- 6 235 501

## Description

L'invention concerne un dispositif de broyage d'un échantillon de matière molle, par exemple biologique, en particulier d'un échantillon de relativement grand volume.

On connaît des broyeurs à plateau oscillant dans lesquels des tubes de petit volume portés par le plateau et contenant des échantillons à broyer et des microbilles, sont soumis à des accélérations alternées intenses qui réalisent un broyage très efficace des échantillons. Ces tubes sont toutefois limités en volume et en masse, les accélérations utilisées ne permettant pas de dépasser une charge de quelques grammes par tube. On connait également des broyeurs tels que décrits dans le document WO 2006/076819A.

Les autres broyeurs connus sont également limités à des volumes utiles inférieurs à 0,1 ou 0,2 litre et il n'existe pas sur le marché de broyeurs pouvant fonctionner dans des conditions voulues de sécurité avec des consommables ayant des volumes utiles de 0,5 litre ou davantage.

L'invention a notamment pour but de répondre à ce besoin.

Elle propose à cet effet un dispositif de broyage selon l'objet de la revendication 1.

Dans le dispositif selon l'invention, le bocal est un consommable qui peut être fermé par le bouchon immédiatement après le dépôt de l'échantillon et qui n'est plus ouvert ensuite. Il suffit, pour le broyage de l'échantillon, de relier l'extrémité supérieure de l'arbre monté dans le bouchon du bocal à des moyens d'entraînement en rotation.

Le broyage de l'échantillon est réalisé par les lames de coupe montées à l'extrémité inférieure de l'arbre.

Selon une autre caractéristique de l'invention, le bouchon du bocal comprend un passage traversant dans lequel est monté un septum destiné au passage d'une aiguille de prélèvement.

Cette aiguille de prélèvement, qui peut être montée dans le septum du bouchon après broyage de l'échantillon et centrifugation, permet d'aspirer une certaine quantité de surnageant, sans qu'il soit nécessaire d'ouvrir le bocal ou de manipuler le bouchon à cet effet.

On limite ainsi les risques de contamination de l'échantillon broyé par l'atmosphère ambiante et de contamination de l'environnement par le contenu du bocal et on assure une sécurité maximale.

Le bocal et son contenu sont destinés à être détruits après prélèvement.

Avantageusement, les moyens d'entraînement en rotation de l'arbre sont déplaçables sur les moyens de support du bocal entre une position inactive où ils sont séparés de l'arbre guidé dans le bouchon et une position active où ils sont reliés à cet arbre pour son entraînement en rotation. Le bouchon du bocal comprend un passage axial traversant aux extrémités duquel sont montés deux paliers lisses de guidage de l'arbre en rotation, l'extrémité inférieure de ce passage traversant étant équipée d'un joint d'étanchéité coopérant avec l'arbre et l'extrémité supérieure du passage comprenant une butée axiale de guidage de l'arbre.

Le bouchon équipé de l'arbre et du septum est vissé sur l'extrémité supérieure du bocal, qui est reçue dans une gorge annulaire du bouchon au fond de laquelle est monté un joint d'étanchéité.

Selon une autre caractéristique de l'invention, les moyens de support et d'immobilisation du bocal comprennent une enceinte calorifugée ouverte à son extrémité supérieure et garnie intérieurement d'un manchon cylindrique souple ayant une forme complémentaire de celle du bocal, ce manchon étant raccordé à des moyens de circulation d'un fluide froid sous pression.

Le fond de l'enceinte est avantageusement recouvert d'un disque ou coussin souple de support du bocal, ce disque ou coussin étant également raccordé aux moyens de circulation de fluide froid sous pression.

On réalise ainsi un support du bocal qui permet d'amortir les vibrations et qui assure un maintien du bocal et de son contenu à une température souhaitée.

L'invention concerne également un ensemble selon la revendication 11.

Ce bocal est de préférence réalisé en matériau plastique translucide ou transparent tel que du polypropylène ou du polyéthylène, et a un volume compris entre 0,2 et 1,5 litre.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
- la figure 1 est une vue schématique en coupe axiale d'un dispositif de broyage selon l'invention ;
- la figure 2 est une vue en coupe transversale de ce dispositif ;
- la figure 3 est une vue en coupe axiale et à plus grande échelle d'un bocal selon l'invention ;
- la figure 4 représente schématiquement des moyens de support et maintien en température du bocal selon l'invention.

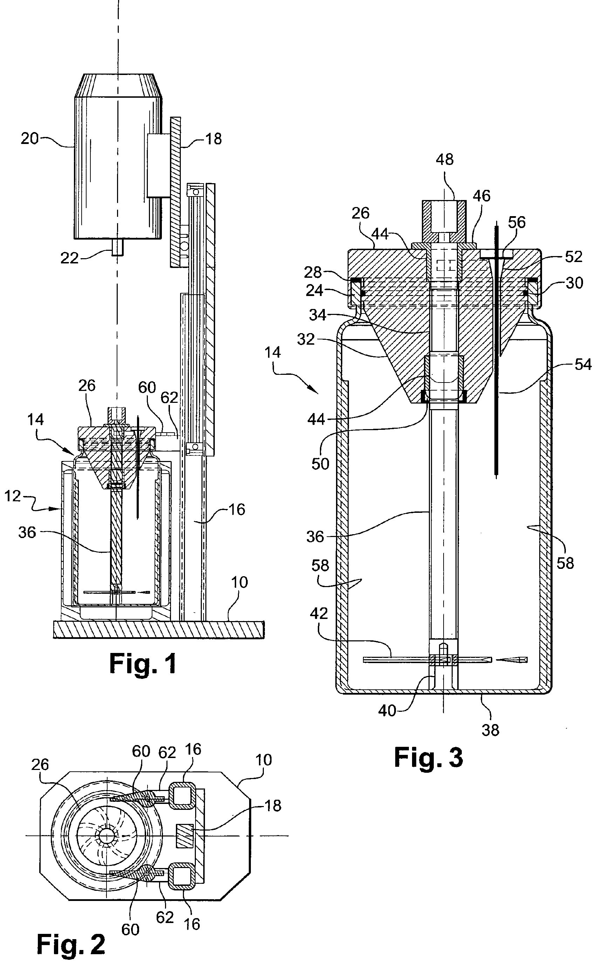

Le dispositif représenté en figure 1 comprend essentiellement une plaque de base 10 portant des moyens 12 de support et d'immobilisation d'un bocal 14 destiné à contenir un échantillon à broyer, la plaque 10 portant également deux montants verticaux 16 de support de moyens 18 de guidage en translation d'un moteur électrique 20 dont l'arbre de sortie vertical 22 est placé au-dessus du bocal 14 et orienté vers celui-ci.

Comme on le voit mieux sur la figure 3, le bocal 14 est un récipient cylindrique en matériau plastique transparent ou translucide, tel par exemple que du polypropylène ou du polyéthylène, qui a un volume d'environ 1 litre dans l'exemple représenté, et dont la forme et le volume sont compatibles avec une nacelle de centrifugeuse. L'extrémité supérieure filetée 24 du bocal est reçue dans une gorge annulaire périphérique d'un bouchon 26 réalisé également, de préférence, en un matériau plastique tel que du polyéthylène ou du polypropylène.

La paroi cylindrique externe de la gorge annulaire du bouchon 26 est filetée pour permettre le vissage du bouchon sur le bocal, un joint d'étanchéité 28 étant placé au fond de cette gorge et comprimé par l'extrémité supérieure du bocal quand le bouchon 26 est vissé complètement sur le bocal.

L'étanchéité de cette fermeture est complétée par un autre joint d'étanchéité 30, monté dans une rainure annulaire de la paroi cylindrique interne de la gorge annulaire du bouchon, ce joint 30 coopérant avec la face cylindrique interne de l'extrémité supérieure 24 du bocal.

Comme représenté, le bouchon 26 peut comprendre une partie inférieure centrale tronconique 32 qui est destinée à s'étendre à l'intérieur du bocal 14 sur une certaine longueur et qui comporte un passage axial traversant 34 dans lequel est monté et guidé un arbre 36 qui s'étend jusqu'au voisinage du fond 38 du bocal 14 et qui peut être centré sur une pointe cylindrique 40 formée en saillie sur le fond du bocal.

L'extrémité inférieure de l'arbre 36 porte des lames de coupe 42 d'un type classique qui sont par exemple incurvées en arc de cercle.

La partie supérieure de l'arbre 36 est guidée en rotation dans le passage 34, au moyen de deux paliers lisses 44 montés aux extrémités de ce passage et d'une butée axiale 46 montée à l'extrémité supérieure du passage 34.

L'extrémité supérieure de l'arbre 36, qui est extérieure au bouchon 26, comporte un embout 48 de liaison à l'arbre de sortie 22 du moteur électrique 20.

Un joint d'étanchéité 50 est monté à l'extrémité inférieure du passage 34, sous le palier lisse 44 et coopère avec la surface cylindrique de l'arbre 36.

Un autre passage traversant 52 est formé dans le bouchon 26, parallèlement au passage axial 34 pour recevoir une aiguille de prélèvement 54 dont l'extrémité inférieure s'étend sur une certaine longueur à l'intérieur du bocal 14, cette aiguille 54 traversant à étanchéité un septum 56 monté dans l'extrémité supérieure élargie du passage 52.

Des nervures verticales 58 sont formées en saillie sur la surface cylindrique interne du bocal 14, pour s'opposer à la mise en rotation de l'échantillon dans le bocal lors du broyage.

Le dispositif de broyage selon l'invention comprend encore deux pattes ou pinces 60 de centrage du bocal 14 sur l'axe de l'arbre 22 du moteur 20, ces pattes ou pinces 60 étant montées pivotantes sur des bras horizontaux 62 portés par les montants verticaux 16 de façon à ce que leurs extrémités libres viennent s'appuyer ou s'engager sur deux points diamétralement opposés de la périphérie du bouchon 26, comme représenté schématiquement en figure 2, et caler ainsi le bouchon 26 et le bocal sur l'axe de l'arbre du moteur 20.

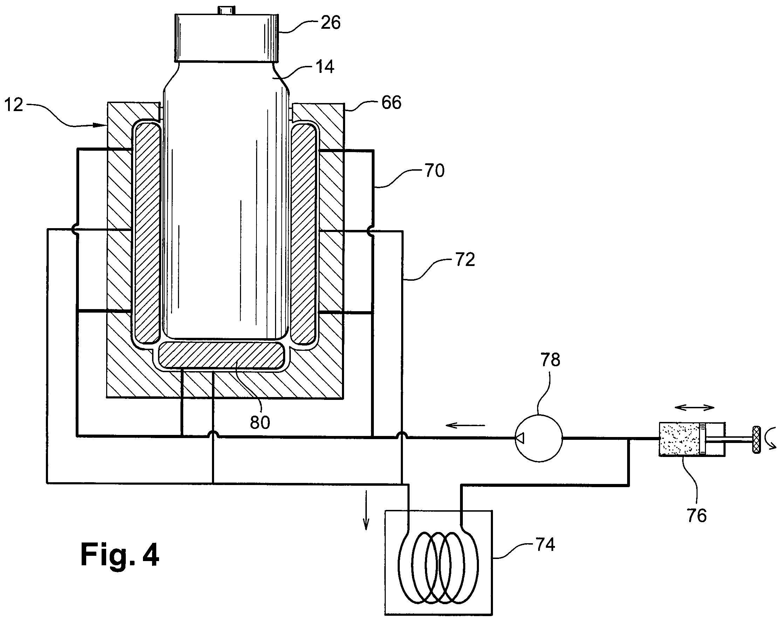

Les moyens 12 de support et d'immobilisation du bocal 14 sont représentés plus en détail en figure 4 et comprennent essentiellement une enceinte 66 calorifugée ou réalisée en matériau thermiquement isolant dont l'extrémité supérieure ouverte permet l'introduction du flacon 14 et qui contient un manchon cylindrique 68 en matériau souple s'étendant sur sensiblement toute la hauteur de l'enceinte 66 et du bocal 14, ce manchon 68 étant raccordé à un circuit d'amenée 70 et à un circuit de retour 72 d'un fluide froid sous pression reliés par des moyens 74 de refroidissement du fluide, le circuit d'amenée 70 comprenant des moyens 76 de mise en pression du fluide et de compensation des dilatations et une pompe 78 de circulation de ce fluide.

Un coussin ou disque circulaire gonflable 80 est monté sur le fond de l'enceinte 66 pour supporter le fond du bocal 14 et est également raccordé aux circuits 70 et 72 d'amenée et de retour du fluide froid.

Le manchon 68 est dimensionné pour s'appliquer étroitement sur la paroi cylindrique du bocal 14 et sur la paroi interne de l'enceinte 66 quand il est rempli de fluide sous pression, ce qui assure un transfert thermique optimal entre le fluide froid et le bocal 14 et permet également de bien caler le bocal 14 dans l'enceinte 66 et d'amortir les vibrations.

Typiquement, le fluide de refroidissement utilisé est de l'eau et permet de maintenir la température de l'échantillon biologique broyé dans le bocal 14 à une valeur de quelques degrés Celsius au-dessus ou au-dessous de zéro. Le dispositif 76 de mise en pression et de compensation des dilatations est un dispositif mécanique très simple comme représenté schématiquement en figure 4, et est par exemple du type à vis ou à tige et à piston.

Le dispositif de broyage selon l'invention est utilisé de la façon suivante :
Chaque bocal 14 est fourni conditionné dans un emballage stérile et équipé d'un bouchon 26 muni d'un arbre porte-couteaux 36 et d'un septum 56.

Il suffit, pour l'utiliser, d'ouvrir le conditionnement stérile, de dévisser le bouchon 26, de placer un échantillon biologique à broyer dans le bocal 14 et de remettre le bouchon 26 en place, en le vissant complètement sur l'extrémité filetée du bocal 14.

Par exemple, l'échantillon biologique à broyer peut être un embryon de poulet dans lequel on a inoculé un virus en vue du développement d'anticorps que l'on prélèvera après broyage et centrifugation.

Après vissage du bouchon 26 sur le bocal, on place le bocal dans l'enceinte 66 des moyens 12 de support et d'immobilisation, on ferme les pinces 60 sur le bouchon 26, on descend le moteur 20 vers le bocal pour engager l'extrémité de l'arbre 22 dans l'embout 48 du bouchon, et on alimente le moteur 20 pour entraîner en rotation les couteaux 42 portés par l'arbre 36, la vitesse de rotation de cet arbre étant typiquement comprise entre 6000 et 12000 tours par minute. La durée du broyage peut varier de quelques secondes à quelques minutes selon les cas.

La température de l'échantillon est maintenue en dessous de 8 ou 10°C par circulation d'un liquide froid dans le manchon 68 et dans le coussin 80.

Après broyage, le bocal 14 fermé par le bouchon 26 est placé dans une centrifugeuse pour séparer le solide du surnageant à l'intérieur du bocal. L'aiguille 54 introduite dans le bocal à travers le septum 56 du bouchon 26 permet de prélever une certaine quantité du surnageant dans le bocal 14.

Ensuite, le bocal 14 toujours fermé par son bouchon 26 équipé de l'arbre 36 est mis de côté pour être détruit.

Le dispositif selon l'invention présente un certain nombre d'avantages importants par rapport à la technique actuelle :
- il utilise une technologie de broyage qui est confirmée et donne satisfaction,
- il offre une garantie totale d'asepsie,
- toutes les opérations de transvasement intermédiaire, de nettoyage, de stérilisation et de conditionnement exécutées dans la technique antérieure sont supprimées,
- son utilisation par les opérateurs est simple et ne pose pas de problème,
- le volume du bocal 14 peut être déterminé à volonté entre 0,1 ou 0,2 litre et 1,5 ou 2 litres notamment.

## Revendications

1. Dispositif de broyage d'un échantillon biologique, **caractérisé en ce qu'**il comprend des moyens (10, 12) de support et d'immobilisation d'un bocal (14) destiné à contenir l'échantillon et fermé à son extrémité supérieure par un bouchon (26) équipé de moyens (44) de guidage en rotation d'un arbre (36) qui s'étend à l'intérieur du bocal et porte des lames de coupe (42) à son extrémité inférieure, l'extrémité supérieure de cet arbre étant extérieure au bouchon (26) et comprenant des moyens (48) de liaison à des moyens (20, 22) d'entraînement en rotation porté par les moyens de support du bocal et dans lequel les moyens de support comprennent un manchon cylindrique souple ayant une forme complémentaire de celle du bocal et raccordé à des moyens (70, 72) de circulation d'un fluide froid sous pression.

2. Dispositif selon la revendication 1, **caractérisé en ce que** les moyens (20, 22) d'entraînement en rotation sont déplaçables sur les moyens de support (10, 12, 16) entre une position inactive où ils sont séparés de l'arbre guidé dans le bouchon du bocal et une position active où ils sont reliés à cet arbre pour son entraînement en rotation.

3. Dispositif selon la revendication 1 ou 2, **caractérisé en ce que** le bouchon (26) du bocal comprend un passage axial traversant (34) aux extrémités duquel sont montés deux paliers lisses (44) de guidage de l'arbre (36) en rotation.

4. Dispositif selon la revendication 3, **caractérisé en ce que** l'extrémité inférieure du passage traversant (34) est équipé d'un joint d'étanchéité (50) coopérant avec l'arbre.

5. Dispositif selon la revendication 3 ou 4, **caractérisé en ce que** l'extrémité supérieure du passage traversant (34) comprend une butée axiale (46) de guidage de l'arbre (36).

6. Dispositif selon l'une des revendications 3 à 5, **caractérisé en ce que** le bouchon (26) comprend un second passage traversant (52) dans lequel est monté un septum (56) pour le passage d'une aiguille de prélèvement (54).

7. Dispositif selon l'une des revendications précédentes, **caractérisé en ce que** le bouchon (26) est vissé sur l'extrémité supérieure filetée (24) du bocal.

8. Dispositif selon l'une des revendications précédentes, **caractérisé en ce que** les moyens de support et d'immobilisation du bocal comprennent une enceinte (66) ouverte à son extrémité supérieure et garnie intérieurement du manchon cylindrique souple (68) ayant une forme complémentaire de celle du bocal, ce manchon étant raccordé à des moyens (70, 72) de circulation d'un fluide froid sous pression.

9. Dispositif selon la revendication 8, **caractérisé en ce que** le fond de l'enceinte (66) est recouvert d'un disque ou d'un coussin souple (80) de support du bocal, ce disque ou coussin étant raccordé aux moyens (71, 72) de circulation d'un fluide froid sous pression.

10. Dispositif selon l'une des revendications précédentes, **caractérisé en ce qu'**il comprend des moyens à pattes pivotantes (60) de centrage du bocal (14) et du bouchon (26) par rapport aux moyens {20, 22) d'entraînement de l'arbre (36) en rotation.

11. Ensemble comprenant un dispositif de broyage selon l'une quelconque des revendications précédentes et un bocal destiné à contenir une échantillon et fermé à son extrémité supérieure par un bouchon (26) équipé de moyens (44) de guidage en rotation d'un arbre (36) qui s'étend à l'intérieur du bocal et porte des lames de coupe (42) à son extrémité inférieure.

12. Ensemble selon la revendication précédente dans lequel le bouchon de fermeture (26) est formé avec un second passage traversant (52) équipé d'un septum (56), comprend une partie inférieure centrale tronconique (32) destinée à s'étendre à l'intérieur du bocal et un premier passage traversant (34) équipé de paliers de guidage de l'arbre (36) porte-couteaux.

13. Ensemble selon l'une quelconque des deux revendications précédentes dans lequel le bocal est réalisé en matériau plastique translucide ou transparent tel que du propylène ou du polyéthylène et a un volume compris entre 0,2 et 1,5 litre.

## Patentansprüche

1. Vorrichtung zum Zerkleinern einer biologischen Probe, **dadurch gekennzeichnet, dass** sie Mittel (10, 12) zum Stützen und Immobilisieren eines Gefäßes (14) umfasst, das dazu bestimmt ist, die Probe zu enthalten, und an seinem oberen Ende durch einen Stopfen (26) verschlossen ist, der mit Mitteln (44) zur Drehführung einer Welle (36) ausgerüstet ist, die sich im Inneren des Gefäßes erstreckt, und an ihrem unteren Ende Schneidklingen (42) trägt, wobei sich das obere Ende dieser Welle außerhalb des Stopfens (26) befindet und Mittel (48) zum Verbinden mit Mitteln (20, 22) zum Drehantrieb umfasst, die von den Mitteln zum Stützen des Gefäßes getragen werden, und wobei die Mittel zum Stützen eine biegsame zylindrische Manschette umfassen, die eine ergänzende Form zu jener des Gefäßes aufweist, und an Mittel (70, 72) zum Zirkulieren eines unter Druck stehenden kalten Fluids angeschlossen ist.

2. Vorrichtung nach Anspruch 1, **dadurch gekennzeichnet, dass** die Mittel (20, 22) zum Drehantrieb auf den Stützmitteln (10, 12, 16) zwischen einer inaktiven Position, in der sie von der geführten Welle in dem Stopfen des Gefäßes getrennt sind, und einer aktiven Position verschiebbar sind, in der sie mit dieser Welle zu deren Drehantrieb verbunden sind.

3. Vorrichtung nach Anspruch 1 oder 2, **dadurch gekennzeichnet, dass** der Stopfen (26) des Gefäßes einen durchgehenden axialen Durchlass (34) umfasst, an dessen Enden zwei Gleitlager (44) zur Drehführung der Welle (36) montiert sind.

4. Vorrichtung nach Anspruch 3, **dadurch gekennzeichnet, dass** das untere Ende des durchgehenden Durchlasses (34) mit einer Dichtung (50) ausgerüstet ist, die mit der Welle zusammenwirkt.

5. Vorrichtung nach Anspruch 3 oder 4, **dadurch gekennzeichnet, dass** das obere Ende des durchgehenden Durchlasses (34) einen axialen Anschlag (46) zum Führen der Welle (36) umfasst.

6. Vorrichtung nach einem der Ansprüche 3 bis 5, **dadurch gekennzeichnet, dass** der Stopfen (26) einen zweiten durchgehenden Durchlass (52) umfasst, in dem ein Septum (56) für den Durchlass einer Entnahmenadel (54) montiert ist.

7. Vorrichtung nach einem der vorstehenden Ansprüche, **dadurch gekennzeichnet, dass** der Stopfen (26) auf das obere Ende mit Gewinde (24) des Gefäßes geschraubt ist.

8. Vorrichtung nach einem der vorstehenden Ansprüche, **dadurch gekennzeichnet, dass** die Mittel zum Stützen und Immobilisieren des Gefäßes eine Einschließung (66) umfasst, der an ihrem oberen Ende offen ist, und im Inneren mit der biegsamen zylindrischen Manschette (68) versehen ist, die eine ergänzende Form zu jener des Gefäßes aufweist, wobei diese Manschette an Mittel (70, 72) zum Zirkulieren eines unter Druck stehenden kalten Fluids angeschlossen ist.

9. Vorrichtung nach Anspruch 8, **dadurch gekennzeichnet, dass** der Boden der Einschließung (66) mit einer Scheibe oder einem biegsamen Kissen (80) zum Stützen des Gefäßes abgedeckt ist, wobei diese Scheibe oder dieses Kissen an die Mittel (71, 72) zum Zirkulieren eines unter Druck stehenden kalten Fluids angeschlossen ist.

10. Vorrichtung nach einem der vorstehenden Ansprüche, **dadurch gekennzeichnet, dass** sie Mittel mit Schwenklaschen (60) zum Zentrieren des Gefäßes (14) und des Stopfens (26) in Bezug auf die Mittel (20, 22) zum Drehantrieb der Welle (36) umfasst.

11. Baugruppe, eine Vorrichtung zum Zerkleinern nach einem der vorstehenden Ansprüche, und ein Gefäß umfassend, das dazu bestimmt ist, eine Probe zu enthalten, und an seinem oberen Ende durch einen Stopfen (26) verschlossen ist, der mit Mitteln (44) zur Drehführung einer Welle (36) ausgerüstet ist, die sich im Inneren des Gefäßes erstreckt, und an ihrem unteren Ende Schneidklingen (42) trägt.

12. Baugruppe nach dem vorstehenden Anspruch, wobei der Schließstopfen (26) mit einem zweiten durchgehenden Durchlass (52) gebildet ist, der mit einem Septum (56) ausgerüstet ist, einen unteren mittleren kegelstumpfförmigen Teil (32) umfasst, der dazu bestimmt ist, sich im Inneren des Gefäßes zu erstrecken, und einen ersten durchgehenden Durchlass (34), der mit Lagern zum Führen der Messerträgerwelle (36) ausgerüstet ist.

13. Baugruppe nach einem der beiden vorstehenden Ansprüche, wobei das Gefäß aus einem durchscheinenden oder durchsichtigen Kunststoffmaterial, wie Polypropylen oder Polyethylen gefertigt ist, und ein Volumen aufweist, das zwischen 0,2 und 1,5 Litern enthalten ist.

## Claims

1. Device for grinding a biological sample, **characterised in that** it comprises means (10, 12) for supporting and immobilising a jar (14) intended to contain the sample and closed at the upper end thereof by a cap (26) equipped with means (44) for guiding a shaft (36) in rotation that extends inside the jar and carries cutting blades (42) at the lower end thereof, the upper end of this shaft being outside the cap (26) and comprising means (48) for connecting to means (20, 22) for driving in rotation caried by the means for supporting the jar and wherein the means for supporting comprise a flexible cylindrical sleeve that has a shape complementary with that of the jar and connected to means (70, 72) for circulating a cold fluid under pressure.

2. Device according to claim 1, **characterised in that** the means (20, 22) for driving in rotation can be displaced on the means for supporting (10, 12, 16) between an inactive position wherein they are separated from the shaft guided in the cap of the jar and an active position wherein they are connected to this shaft for the driving in rotation thereof.

3. Device according to claim 1 or 2, **characterised in that** the cap (26) of the jar comprises an axial through-passage (34) at the ends of which are mounted two slide bearings (44) for guiding the shaft (36) in rotation.

4. Device according to claim 3, **characterised in that** the lower end of the through-passage (34) is equipped with a seal (50) that cooperates with the shaft.

5. Device according to claim 3 or 4, **characterised in that** the upper end of the through-passage (34) comprises an axial abutment (46) for guiding the shaft (36).

6. Device according to one of claims 3 to 5, **characterised in that** the cap (26) comprises a second through-passage (52) wherein a septum (56) is mounted for a sampling needle (54) to pass through.

7. Device according to one of the preceding claims, **characterised in that** the cap (26) is screwed onto the threaded upper end (24) of the jar.

8. Device according to one of the preceding claims, **characterised in that** the means for supporting and immobilising the jar comprise an enclosure (66) open at the upper end thereof and lined interiorly with the flexible cylindrical sleeve (68) having a shape complementary with that of the jar, this sleeve being connected to means (70, 72) for circulating a cold fluid under pressure.

9. Device according to claim 8, **characterised in that** the bottom of the enclosure (66) is covered with a disc or with a flexible cushion (80) for supporting the jar, this disc or cushion being connected to the means (71, 72) for circulating a cold fluid under pressure.

10. Device according to one of the preceding claims, **characterised in that** it comprises means with pivoting tabs (60) for centring the jar (14) and the cap (26) with respect to the means (20, 22) for driving the shaft (36) in rotation.

11. Assembly comprising a device for grinding according to any preceding claim and a jar intended to contain a sample and closed at the upper end thereof by a cap (26) equipped with means (44) for guiding a shaft (36) in rotation that extends inside the jar and carries the cutting blades (42) at the lower end thereof.

12. Assembly according to the preceding claim wherein the closing cap (26) is formed with a second through-passage (52) equipped with a septum (56), comprises a truncated central lower portion (32) intended to extend inside the jar and a first through-passage (34) equipped with bearings for guiding the knife-holder shaft (36).

13. Assembly according to any of the two preceding claims wherein the jar is made from a translucent or transparent plastic material such as propylene or polyethylene and has a volume comprised between 0.2 and 1.5 litre.
